# EUROPÄISCHE PATENTSCHRIFT

(11) **EP 1 084 763 B1**
(45) Veröffentlichungstag und Bekanntmachung des Hinweises auf die Patenterteilung: **27.12.2006**
(21) Anmeldenummer: 00119964.5
(22) Anmeldetag: 14.09.2000
(51) Int. Cl.: B05B 11/00, A61M 15/00, B05B 11/02, A61M 5/315

(54) **Spender zum ggf. zerstäubten Ausbringen eines insbesondere flüssigen Mediums aus einem Behältnis**
Dispenser for dispensing, in particular spraying, a fluid from a container
Distributeur, notamment pour pulvériser un produit fluide contenu dans un réservoir

(30) Priorität: 15.09.1999 DE 19944209
(43) Veröffentlichungstag der Anmeldung: 21.03.2001
(60) Teilanmeldung: 06023416.8
(73) Patentinhaber: Ing. Erich Pfeiffer GmbH, 78315 Radolfzell (DE)
(72) Erfinder: Greiner-Perth, Jürgen, 78244 Gottmadingen (DE)
(74) Vertreter: Patentanwälte Ruff, Wilhelm, Beier, Dauster & Partner

(56) Entgegenhaltungen:
- WO-A-96/24439
- DE-A- 4 008 068
- US-A- 4 962 868
- US-A- 5 284 132

## Beschreibung

Die Erfindung betrifft Spender zum ggf. zerstäubten Ausbringen eines insbesondere flüssigen Mediums aus einem Behältnis.

Ein derartiger Spender ist beispielsweise aus der EP 0 334 349 A1 bekannt, bei der das Ausbringen des Mediums durch manuelle Betätigung eines Betätigungselements erfolgt. Dabei ist die Betätigung in eine Folge von Teilbetätigungen untergliedert, wobei bei jeder Teilbetätigung eine definierte Teilcharge des Mediums ausgebracht wird. Um zu verhindern, daß zwei aufeinander folgende Teilbetätigungen ununterbrochen hintereinander durchgeführt werden, ist es aus dieser Druckschrift auch bekannt, daß zwischen zwei Teilbetätigungen des Betätigungselementes eine Umschaltbetätigung erforderlich ist.

Derartige Spender finden insbesondere Anwendung, wenn ein Medikament oder ein Impfstoff auf die Nasenschleimhäute eines Patienten appliziert werden soll. Ein zerstäubtes Ausbringen des Mediums hat dabei den Vorteil, daß die Absorption des Wirkstoffs durch den Patienten leichter erfolgt. Ein Beispiel, bei dem der Spender auch geeignet sein soll, durch den Patienten selbst genutzt zu werden, sind auf die Nasenschleimhäute zu applizierende Migränemittel.

Das Medium muß dabei in üblicherweise zwei, jedenfalls aber in einer geringen Anzahl, von Teilchargen definierter - meist gleicher - Größe aus dem Spender ausgebracht werden. Im Interesse einer großen Benutzungssicherheit wird dabei vorgeschlagen, den Betätigungsweg des Betätigungselements bewußt zu unterbrechen, damit nicht versehentlich in einer durchgehenden Betätigung des Betätigungselements mehr als eine Teilcharge auf einmal ausgebracht wird. Um dies sicherzustellen, ist zwischen den Teilbetätigungen des Betätigungselements eine Umschaltbetätigung erforderlich.

Die Durchführung einer solchen Umschaltbetätigung erfordert jedoch ein Absetzen des Spenders vom Applikationsort und steht einer Einhandbetätigung des Spenders entgegen.

Eine Einhandbetätigung eines solchen Spenders ist jedoch insbesondere dann wünschenswert, wenn der Benutzer die zweite Hand zur Fixierung des Ausbringungsortes - beispielsweise zur Fixierung des Kopfes eines Patienten, insbesondere eines Kinde - benötigt, die somit nicht zur Betätigung des Spenders selbst zur Verfügung steht. Ein Absetzen des Spenders und ein Lösen der Fixierung des Applikationsortes zur Durchführung der Umschaltbetätigung und eine anschließende Neufixierung des Applikationsortes und ein neues Ansetzen des Spenders wäre sehr umständlich.

Aus der DE 40 08 068 A1 ist ein Spender bekannt, bei dem mehrere Teilhübe aufeinander folgend ausführbar sind, die am jeweiligen Ende durch Anschlag begrenzt sind. Die für den folgenden Teilhub notwendige Umschaltbetätigung wird entweder manuell durch Verdrehung zweier Spenderteile gegeneinander oder durch eine Kulissenführung bewirkt, die schon während des vorangehenden Teilhubes eine Verdrehung der beiden Spenderteile gegeneinander bewirkt und danach unter Wirkung einer axial, also in Richtung der Teilhub-Betätigung, wirkenden Feder und durch eine zweite Kulissenfläche in eine Kulissenbahn für den folgenden Teilhub gebracht wird.

### AUFGABE UND LÖSUNG

Aufgabe der Erfindung ist es daher, einen derartigen Spender dahingehend weiterzubilden, daß eine Einhandbetätigung, bei der sichergestellt ist, daß ein Ausbringen von mehr als einer Teilcharge in einer Betätigung zuverlässig verhindert ist, selbsttätig ermöglicht wird.

Diese Aufgabe wird durch die kennzeichnenden Merkmale des Anspruchs 1 gelöst.

Bei einem erfindungsgemäßen Spender zum ggf. zerstäubten Ausbringen eines insbesondere flüssigen Mediums aus einem Behältnis erfolgt die Betätigung des Spenders in einer Folge von manuellen Teilbetätigungen eines Betätigungselements. Bei jeder der Teilbetätigungen des Betätigungselements wird aus dem Spender eine definierte Teilcharge ausgebracht. Zwischen zwei Teilbetätigungen des Betätigungselements erfolgt dabei eine Umschaltbetätigung. Diese Umschaltbetätigung erfolgt nach Durchführung einer Teilbetätigung jeweils selbsttätig.

Zur Durchführung der Umschaltbetätigung sind Kraftspeicher vorgesehen, die während der Durchführung einer Teilbetätigung vorgespannt werden. Es ist insbesondere vorteilhaft, die Umschaltbetätigung dann durchzuführen, wenn das Ende der Teilbetätigung dadurch festgestellt wird, daß die Betätigungskraft die zur Durchführung der Teilbetätigung erforderlich ist, nicht mehr auf das Betätigungselement einwirkt.

Gemäß weiteren Ausgestaltungen der Erfindung ist das Behältnis in einem Gehäuse angeordnet, wobei zwischen Gehäuse und Betätigungselement während jeder Teilbetätigung eine Relativbewegung stattfindet, wobei diese Relativbewegung durch eine Kulissenführung festgelegt ist. Die Kulissenführung erfolgt insbesondere durch eine Kulissenbahn, in der ein Gleitstein geführt ist. Gemäß einer bevorzugten Ausführungsform ist zwischen Gehäuse und Behältnis eine Hülse angeordnet, wobei die Hülse entweder zum Gehäuse oder zum Behältnis lagefest bzgl. einer Teilbetätigung des Betätigungselements jedoch in Richtung der Umschaltbetätigung relativ beweglich hierzu angeordnet ist. Die Hülse weist dabei entweder die Kulissenbahn oder aber den in der Kulissenbahn geführten Gleitstein auf. Weitere bevorzugte Ausbildungen der Kulissenführung sind in den weiteren Unteransprüchen aufgeführt. Diese betreffen insbesondere die Gestaltung der Kulissenbahn selbst sowie die Ausbildung von Kraftspeichern an der Kulissenbahn.

Neben den Unteransprüchen ist die Erfindung anhand der in der Zeichnung dargestellten Ausführungsbeispiele näher erläutert; dabei zeigt:
- Fig. 1: ein Schnittbild durch einen ersten erfindungsgemäßen Spender, bei dem eine Hülse mit Kulissenbahn lagefest zu einem das Behältnis aufnehmenden Gehäuse angeordnet ist und
- Fig. 2a, 2b: Seitenansichten einer Hülse zur Verwendung in der Ausführungsform gemäß Fig. 1.

### BESCHREIBUNG EINES AUSFÜHRUNGSBEISPIELS

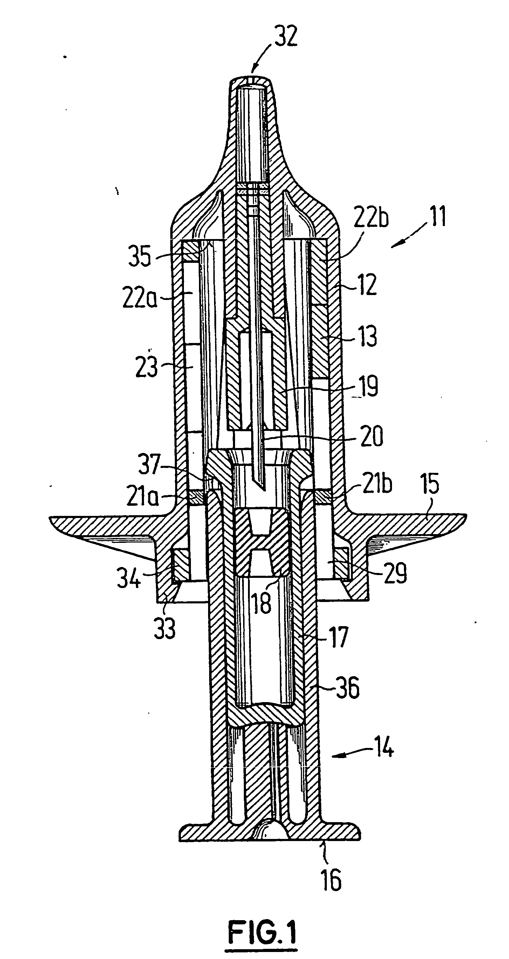

Die Fig. 1 zeigt in Querschnittsdarstellung einen erfindungsgemäßen Spender, der in Schubkolbenbauweise ausgeführt ist. Der Spender 11 besteht aus einem Gehäuse 12, der im Gehäuse drehbar gehaltenen Hülse 13, und dem Betätigungselement 14, das zugleich als Aufnahme für die Ampulle 17, die als Behältnis für das auszubringende Medium dient, besteht. Die Ampulle 17 ist vor der ersten Betätigung durch einen Stopfen 18 verschlossen. Die Ampulle 17 ist in dem Betätigungselement 14 so gehalten, daß sie bei einer Betätigung des Betätigungselements in Richtung auf das Gehäuse hin mit dem Betätigungselement 14 mitbewegt wird. Während des ersten Betätigungsteilhubes wird zunächst der Stopfen 18 von der Kanüle 20, die einen Ausbringungskanal bis zu der Düse 32 bildet, durchstochen. Anschließend wird der Stopfen 18, der dann als Kolben wirkt, von der im Gehäuse 12 angeordneten Kolbenstange 19 in die Ampulle hineingedrückt, so daß das für das Medium zur Verfügung stehende Volumen der Ampulle verringert wird. Das Medium entweicht über die Kanüle 20 und die Düse 32 und wird so ausgebracht. Aufgrund der Unterteilung des Betätigungsweges, im vorliegenden Fall zwei Teilbetätigungen, wird der Stopfen 18 bei jeder der beiden Teilbetätigungen nur um ein vorgegebenes Maß, vorliegend also nur um die Hälfte des gesamten möglichen Betätigungsweges, in die Ampulle hineingedrückt.

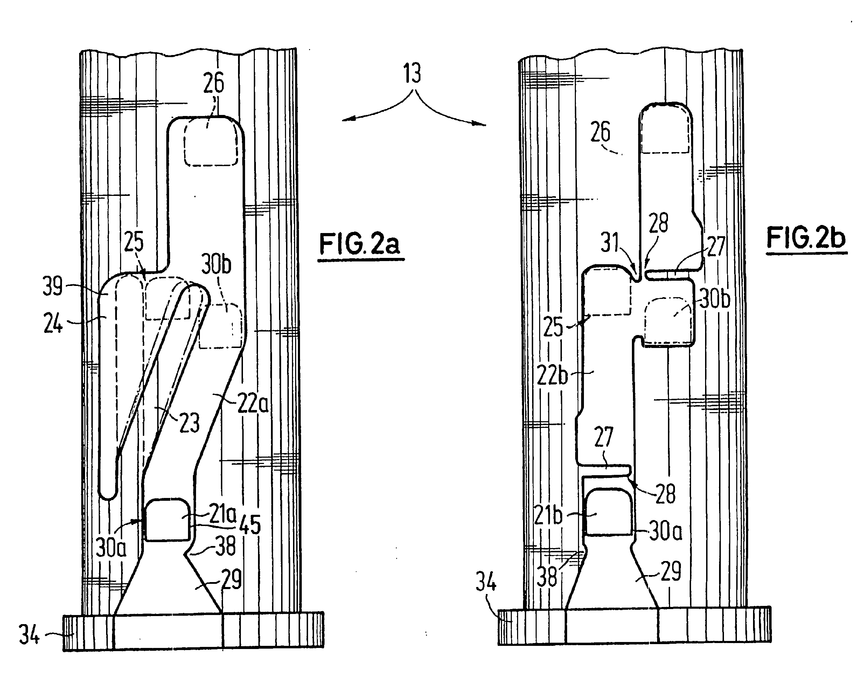

Das Gehäuse 12 des Spenders 11 weist an seinem vorderen Ende die Düse 32 auf, an seinem hinteren Ende die Fingerauflage 15 zur Auflage wenigstens eines Fingers. Die Fingerauflage 15 dient zur Erbringung einer die auf das Betätigungselement 14 einwirkende Betätigungskraft abstützenden Kraft auf das Gehäuse, so daß der Spender während der Betätigung ruhig gehalten ist. Im Gehäuse ist entlang der Gehäusewandung die Hülse 13 angeordnet. Die Hülse 13 stützt sich in der axialen Betätigungsrichtung des Betätigungselements 14 an der Stützkante 35 des Gehäuses 12 ab. An ihrem hinteren Ende weist die Hülse 13 einen Hülsenrand 34 auf, der von der gehäuseseitigen Rastkante 33 so hintergriffen wird, daß die Hülse 13 nicht nach hinten aus dem Gehäuse 12 herausgezogen werden kann. Durch Stützkante 35 und Rastkante 33 ist somit eine Lagerung der Hülse 13 im Gehäuse gebildet, die in Betätigungsrichtung des Betätigungselements 14 eine lagefeste Halterung der Hülse 13 im Gehäuse 12 definiert. Dabei kann die Hülse 13 im Gehäuse 12 um eine in Betätigungsrichtung des Betätigungselements 14 verlaufende Drehachse verdreht werden. Das Betätigungselement 14 weist an seinem hinteren Ende eine Betätigungsfläche 16 auf, die der manuellen Betätigung des Betätigungselements 14 insbesondere mittels des Daumens des Benutzers dient. Im übrigen wird das Betätigungselement 14 im wesentlichen aus einem hohlzylindrischen Schaft 36 gebildet. Dabei weist der hohlzylindrische Schaft 36 einen Außendurchmesser auf, der im wesentlichen dem Innendurchmesser der Hülse 13 entspricht. Der Schaft 36 wird im Gehäuse 12 durch die Hülse 13 geführt. Zur Ausbildung einer Kulissenführung weist die Hülse 13 auf einander gegenüberliegenden Seiten je eine Kulissenbahn auf, wie sie in der Fig. 2a und Fig. 2b näher dargestellt sind. Im Bereich des vorderen Endes des Schaftes 36 des Betätigungselements 14 sind an entsprechenden Stellen Gleitsteine 21a, 21b ausgebildet. In den Schaft 36 wird die Ampulle 17 mit dem darin bevorrateten Medium eingesetzt. Dabei entspricht der Innendurchmesser des Schaftes im wesentlichen dem Außendurchmesser der Ampulle, so daß eine klemmende Halterung der Ampulle 17 im Schaft 36 des Betätigungselements 14 erfolgt. Ferner weist die Ampulle an ihrem vorderen Ende eine Querschnittserweiterung auf, die als Stoßkante 37 in Anlage mit der vorderen Kante des Schaftes 36 gelangen kann, so daß sichergestellt ist, daß die Ampulle 17 bei Betätigung des Betätigungselements 14 in dessen Betätigungsrichtung spielfrei mitgeführt wird.

Die Fig. 2a, 2b zeigen gegenüberliegende Seitenansichten der Hülse 13 mit den Kulissenbahnen 22a, 22b und den jeweils in der Kulissenbahn geführten Gleitsteinen 21a, 21b. Dabei dient die in der Fig. 2a dargestellte Kulissenbahn 22a der Erzeugung der Vorspannung eines Kraftspeichers zur Durchführung der Umschaltbetätigung zwischen den beiden Teilbetätigungen des Betätigungselements während die in Fig. 2b gezeigte Kulissenbahn der Unterteilung des Betätigungsweges des Betätigungselements in zwei Teilbetätigungen dient.

Beide Kulissenbahnen weisen im Bereich des Hülsenrandes 34 einen Einführtrichter 29 zur einfachen Einführung der Gleitsteine 21a, 21b in die jeweilige Kulissenbahn 22a, 22b auf. Ferner sind jeweils Druckpunktmittel 38 im Endbereich der Einführtrichter 29 ausgebildet, die ein Herausgleiten der Gleitsteine 21a, 21b nach hinten aus der Kulissenbahn 22a bzw. 22b verhindern. Somit wird eine Verlustsicherung für das Betätigungselement 14 gebildet.

Die in Fig. 2a dargestellte Kulissenbahn weist einen Federarm 23 auf, der in den Bewegungsraum des Gleitsteins 21a hineinragt und von diesem bei der ersten Teilbetätigung unter Erzeugung einer Vorspannung in den, im Bereich der Hülse materialfreien Verdrängungsraum 39 hineingedrückt wird. Der Federarm 23 besteht vorzugsweise aus dem gleichen Material wie die Hülse selbst und kann als dünner Materialsteg in dem materialfreien Bereich der Kulissenbahn 22a ausgebildet sein. Vorzugsweise wird die Hülse 13 aus Kunststoff hergestellt, wobei beim Spritzgießen der Hülse 13 im selben Spritzvorgang gleich der Federarm 23 erzeugt werden kann.

Das Verdrängen des Federarms 23 aus dem Bewegungsraum des Gleitsteins 21a wird dadurch erforderlich, daß die Bahnkurve der in Fig. 2b dargestellten gegenüberliegend auf der Hülse angeordneten zweiten Kulissenbahn 22b über die gesamte Länge einer ersten Teilbetätigung hinweg geradlinig verläuft. Dadurch wird verhindert, daß der Gleitstein 21a geführt durch den Federarm 23 schon jetzt eine Drehbewegung der Hülse 13 im Gehäuse 12 erzeugt.

Die Fig. 2a zeigt auch eine vorteilhafte Ausführungsform des Gleitsteins 21a. Er ist nur auf seiner Vorderseite verrundet um ein Verteilen in der Kulissenbahn 22a zu vermeiden. Er weist darüber hinaus wenigtens eine Flanke 45 auf, die aufgrund ihrer Längserstreckung parallel zu den linearen Bahnabschnitten der Kulissenbahn eine lange Führung des Gleitsteins 21a in der Kulissenbahn 22a sicherstellt. Auch der in der Fig. 2b dargestellte Gleitstein 21b kann eine entsprechende Form mit einer Flanke 45 aufweisen.

Wie aus der Fig. 2b ersichtlich, ist der Gleitstein 21b in einer Kulissenbahn 22b geführt, die aus zwei linearen Bahnabschnitten besteht, die lediglich um in etwa die Breite der Kulissenbahn zueinander versetzt sind, wobei die Länge der beiden geradlinigen Abschnitte jeweils dem Betätigungsweg einer Teilbetätigung des Betätigungselements 14 entspricht. Dabei überlappen sich die beiden geradlinigen Abschnitte in einem Bereich dessen Länge in etwa dem Durchmesser des Gleitsteins 21b entspricht. Wird das Betätigungselement 14, an dem die Gleitsteine 21a, 21b betätigt, so läuft zunächst der Gleitstein 21b auf das Sperrmittel 27, einen dünnen, an einer Seite der Kulissenbahn 22b abragenden Materialsteg, der über eine Sollbruchstelle 28 mit der gegenüberliegenden Kante der Kulissenbahn 22b verbunden ist und nach Abtrennung an der Sollbruchstelle vollständig aus der Kulissenbahn herausführbar ist, auf. Erst wenn an dem Betätigungselement 14 eine Betätigungskraft aufgebracht wird, die zum Bruch an der Sollbruchstelle 28 führt, wird der das Sperrmittel 27 bildende Materialsteg aus der Kulissenbahn 22b seitlich herausgedrückt. Das Sperrmittel 27 mit seiner Sollbruchstelle 28 stellt somit sicher, daß am Betätigungselement 14 eine derartige Betätigungskraft aufgebracht ist, daß eine vollständige Teilbetätigung des Betätigungselementes in einem zusammenhängenden Betätigungsablauf durchgeführt wird. Der Gleitstein 21b gleitet dann in einer linear verlaufenden, ununterbrochenen Bewegung bis zu der am Ende des ersten linearen Abschnittes der Kulissenbahn 22b ausgebildeten ersten Teilbetätigungsendlage 25. In dieser Lage liegt der in Fig. 2a dargestellte Gleitstein 21a im Bereich des vorderen Endes des Federarms 23, der im dargestellten Beispiel nach links aus dem Bewegungsraum des Gleitsteins 21a herausgedrückt wurde und nun gegen diesen mit entsprechender Hebellänge und der aufgrund der Materialverbiegung erzeugten Kraft gegen den Gleitstein 21a drückt.

Wenn das Betätigungselement 14 selbst nicht zum Gehäuse 12 verdrehbar ist - hier kann die Hemmungswirkung, die durch die im Stopfen 18 befindliche Kanüle 20 sowie durch die am Stopfen und ggf. auch an der Innenseite der Ampulle anliegenden Kolbenstange 19 des Gehäuses 12 entsteht, zu groß sein - wird die Hülse 13 gegenüber dem Betätigungselement 14 mit den daran ausgebildeten Gleitsteinen 21a, 21b aufgrund der im Federarm 23 erzeugten Kraft verdreht. Dies ist nur im Überlappungsbereich der beiden geradlinigen Abschnitte der Kulissenbahn 22b möglich. Um eine saubere Trennung zwischen den beiden Teilbetätigungen zu erreichen, ist am der ersten TeilbetätigungsEndlage 25 entsprechenden Bereich des ersten geradlinigen Abschnittes der Kulissenbahn 22b eine Entlastungsnase 31 ausgebildet, die ein Verdrehen der Hülse 13 bzgl. der Gleitsteine 21a, 21b so lange verhindert, wie nicht das Betätigungselement 14 vollständig von der Betätigungskraft entlastet wurde. Erst danach kann die Entlastungsnase überwunden werden, wobei dabei die Hülse in vernachlässigbarer Weise in Betätigungsrichtung des Betätigungselements 14 verschoben wird.

Durch das Verdrehen der Hülse bzgl. den Gleitsteinen 21a, 21b und den das die Hülse 13 haltenden Gehäuses 12 erreicht der Gleitstein 21b die Betätigungsausgangslage 30b. Durch erneute Betätigung des Betätigungselements 14 gelangt der Gleitstein 21b zunächst wiederum in Anlage an Sperrmittel 27 mit einer Sollbruchstelle 28, die mit einer Mindestbetätigungskraft am Betätigungselement überwunden werden muß. Danach wird der Bewegungsraum für die zweite Teilbetätigung, gebildet durch den zweiten linearen Abschnitt der Bahnkurve der Kulissenbahn 22b freigegeben. Die Kulissenbahn 21a ist dabei, wie in Fig. 2a dargestellt, so ausgebildet, daß sowohl die Drehbewegung als auch die Teilbetätigungsbewegung durch den Gleitstein 21a innerhalb der Bahnkurve der Kulisse 22a freigegeben ist.

Eine das Zurückziehen des Betätigungselements 14 nach Abschluß der Teilbetätigungen verhindernde Rückführung wird durch die in der Fig. 2a dargestellte Bahnkurve der Kulissenbahn 22a in Zusammenwirken mit dem Federarm 23 und der Formgebung des Gleitsteins 21a dadurch erreicht, daß der Gleitstein beim Zurückziehen des Betätigungselements mittels Federarm 23 in den toten Schacht 24 hineinbewegt wird, sobald die Umschaltbetätigung durch das Federelement 23 durchgeführt wurde.

Alternativ ist es auch möglich, die Hülse 13 im Gehäuse 12 auch drehfest anzuordnen. Dann ist darauf zu achten, daß die Hemmung - insbesondere zwischen Stopfen 18 und Kanäle 20 - nicht so groß ist, daß ein Verdrehen der Ampulle 17 samt Betätigungselement 14 in der Hülse 13 aufgrund der durch den Federarm 23 erzeugten Kraft nicht verhindert wird. Eine solche Ausgestaltung hat den Vorteil, daß insbesondere bei einer beispielsweise durch Verrippung rauhen Betätigungsfläche 16, ein Verdrehen von Ampulle 17 und Betätigungselement 14 erst möglich ist, wenn der Benutzer das Betätigungselement losläßt. Das verhindert ebenfalls zuverlässig, daß zwei Teilbetätigungen ohne Unterbrechung hintereinander durchgeführt werden.

## Patentansprüche

1. Spender zum ggf. zerstäubten Ausbringen eines insbesondere flüssigen Mediums aus einem Behältnis (17), wobei das Ausbringen des Mediums durch eine Folge von manuellen Teilbetätigungen eines Betätigungselements (14) in einer Betätigungsrichtung in definierten Teilchargen erfolgt, wobei zwischen zwei Teilbetätigungen des Betätigungselements (14) jeweils eine Umschaltbetätigung durch Relativbewegung vorgesehen ist, die nach Durchführung einer Teilbetätigung selbsttätig erfolgt, wobei während der Teilbetätigung ein Kraftspeicher (23) vorgespannt wird, der nach Abschluß der Teilbetätigung die Umschaltbetätigung bewirkt, **dadurch gekennzeichnet, dass** der Kraftspeicher (23) eine quer zur Betätigungsrichtung wirkende Feder ist.

2. Spender nach Anspruch 1, **dadurch gekennzeichnet, daß** das Betätigungselement (14) durch die Wirkung des Kraftspeichers (23) nach jeder Teilbetätigung in eine Betätigungsausgangslage überführbar ist.

3. Spender nach Anspruch 1 oder 2, **dadurch gekennzeichnet, daß** das Behältnis (17) in einem Gehäuse (12) angeordnet ist, wobei während jeder Teilbetätigung die Relativbewegung zwischen Gehäuse (12) und Betätigungselement (14) stattfindet, und diese Relativdrehung durch eine Kulissenführung festgelegt ist.

4. Spender nach Anspruch 3, **dadurch gekennzeichnet, daß** die Kulissenführung durch wenigstens eine Kulissenbahn (22, 22a, 22b) und darin geführten Gleitstein (21, 21a, 21b) erfolgt.

5. Spender nach Anspruch 4, **dadurch gekennzeichnet, daß** eine Hülse (13) zwischen Gehäuse (12) und Behältnis (17) angeordnet ist, wobei die Hülse (12) entweder die wenigstens eine Kulissenbahn (22, 22a, 22b) oder darin geführten Gleitstein (21, 21a, 21b) aufweist, wobei die Hülse lagefest entweder zum Gehäuse (12) oder zum Behältnis (17) angeordnet ist.

6. Spender nach Anspruch 5, **dadurch gekennzeichnet, daß** die Hülse (13) in Richtung der Teilbetätigung lagefest entweder zum Gehäuse (12) oder zum Behältnis (17) und in Richtung der Umschaltbetätigung relativbeweglich dazu angeordnet ist.

7. Spender nach einem der Ansprüche 3 bis 6, **dadurch gekennzeichnet, daß** die Kulissenführung aus zwei Kulissenbahnen (22a, 22b) mit je einem Gleitstein (21a, 21b) gebildet wird, wobei die erste Kulissenbahn (21a) eine in Betätigungsrichtung den Teilbetätigungen entsprechend gestufte Bahnkurve aufweist, wobei ein in Teilbetätigungen entsprechenden Kulissenbahnabschnitten aus dem Bewegungsraum des Gleitsteins (21a) quer herausschiebbare Federarm (23) als Kraftspeicher hineinragen, wobei am Ende der Teilbetätigung eine Querbewegung des Gleitsteins (21a) aus dem Kulissenbahnabschnitt der erfolgten Teilbetätigung in den Kulissenbahnabschnitt der nachfolgenden Teilbetätigung erfolgt.

8. Spender nach Anspruch7, **dadurch gekennzeichnet, daß** der Federarm (23) derart ausgebildet ist, daß er eine Rückzugssperre für den Gleitstein (21a) bildet.

9. Spender nach einem der Ansprüche 3 bis 8, **dadurch gekennzeichnet, daß** die Kulissenführung aus zwei Kulissenbahnen (22a, 22b) gebildet wird, wobei nur eine der Kulissenbahnen (21a) Federarme (23) aufweist.

10. Spender nach einem der Ansprüche 5 bis 9, **dadurch gekennzeichnet, daß** die Hülse (13) die wenigstens eine Kulissenbahn (22, 22a, 22b) aufweist.

11. Spender nach einem der Ansprüche 3 bis 10, **dadurch gekennzeichnet, daß** die Hülse (13) in Betätigungsrichtung der Teilbetätigungen des Betätigungselements (14) lagefest zum Gehäuse (12) angeordnet und der Gleitstein (21, 21a, 21b) am Betätigungselement (14) ausgebildet ist.

12. Spender nach einem der Ansprüche 3 bis 10, **dadurch gekennzeichnet, daß** die Hülse in Betätigungsrichtung der Teilbetätigungen lagefest zum Behältnis (17) angeordnet und der Gleitstein (21, 21a, 21b) am Gehäuse (12) ausgebildet ist.

13. Spender nach einem der Ansprüche 1 bis 12, **dadurch gekennzeichnet, daß** wenigstens ein Sperrmittel (27) vorgesehen ist, das Teilbetätigungen des Betätigungselements (14) verhindert, soweit nicht eine Mindestbetätigungskraft am Betätigungselement (14) aufgebracht ist.

14. Spender nach einem der vorstehenden Ansprüche, **dadurch gekennzeichnet, daß** das Behältnis die Ampulle (17) eines Schubkolbenzylinders ist, die mit einem Stopfen (18) verschlossen ist, wobei am Gehäuse ein Ausbringungskanal ausgebildet ist, der in einer Ausbringungsöffnung (32) mündet, der an seinem der Ausbringungsöffnung abgewandten Seite in einer Kanüle (20) endet, die während der ersten Teilbetätigung den Stopfen (18) durchsticht, wobei der Stopfen (18) während jeder Teilbetätigung um ein vorgegebenes Maß in Richtung auf den Grund der Ampulle (17) in die Ampulle hineinbewegt wird und ein vorgegebenes Volumenmedium in den Ausbringungskanal verdrängt.

## Claims

1. Dispenser for the optionally atomised discharge of an in particular liquid medium from a container (17), wherein the medium is discharged through a sequence of manual partial operations of an operating member (14) in one operating direction in defined partial batches, wherein between two partial operations of the operating member (14) there is provided in each case a changeover operation by way of relative movement which takes place automatically after a partial operation has been performed, wherein during the partial operation an energy storing device (23) is prestressed and at the end of the partial operation it performs the changeover operation, **characterised in that** the energy storing device (23) is a spring acting transversely with respect to the operating direction.

2. Dispenser as claimed in Claim 1, **characterised in that** the operating member (14) can be transferred after each partial operation into an operating starting position by the action of the energy storing device (23).

3. Dispenser as claimed in Claim 1 or 2, **characterised in that** the container (17) is disposed in a casing (12), wherein during each partial operation the relative movement takes place between the casing (12) and the operating member (14) and this relative rotation is fixed by a sliding link guide.

4. Dispenser as claimed in Claim 3, **characterised in that** the sliding link guide is effected by means of at least one sliding link path (22, 22a, 22b) and a sliding block (21, 21a, 21b) guided therein.

5. Dispenser as claimed in Claim 4, **characterised in that** a sleeve (13) is disposed between the casing (12) and the container (17), wherein the sleeve (12) comprises either the at least one sliding link path (22, 22a, 22b) or the sliding block (21, 21a, 21b) guided therein, wherein the sleeve is disposed in a positionally-stable manner either with respect to the casing (12) or to the container (17).

6. Dispenser as claimed in Claim 5, **characterised in that** the sleeve (13) is disposed in the direction of the partial operation in a positionally-stable manner either with respect to the casing (12) or to the container (17) and in the direction of the changeover operation in such a manner as to be able to move relative thereto.

7. Dispenser as claimed in any one of Claims 3 to 6, **characterised in that** the sliding link guide is formed from two sliding link paths (22a, 22b) having in each case a sliding block (21a, 21b), wherein the first sliding link path (21a) comprises a path curve stepped in accordance with the partial operations in the operating direction, wherein a spring arm (23), which can be slid transversely out of the movement space of the sliding block (21a) in sliding link path portions corresponding to the partial operations, extends as the energy storing device, wherein at the end of the partial operation the sliding block (21a) moves transversely out of the sliding link path portion of the partial operation which has taken place into the sliding link path portion of the following partial operation.

8. Dispenser as claimed in Claim 7, **characterised in that** the spring arm (23) is formed in such a manner that it forms a return barrier for the sliding block (21a).

9. Dispenser as claimed in any one of Claims 3 to 8, **characterised in that** the sliding link guide is formed from two sliding link paths (22a, 22b), wherein only one of the sliding link paths (21a) has spring arms (23).

10. Dispenser as claimed in any one of Claims 5 to 9, **characterised in that** the sleeve (13) comprises the at least one sliding link path (22, 22a, 22b).

11. Dispenser as claimed in any one of Claims 3 to 10, **characterised in that** the sleeve (13) is disposed in a positionally-stable manner with respect to the housing (12) in the operating direction of the partial operations of the operating member (14) and the sliding block (21, 21a, 21b) is formed on the operating member (14).

12. Dispenser as claimed in any one of Claims 3 to 10, **characterised in that** the sleeve is disposed in a positionally-stable manner with respect to the container (17) in the operating direction of the partial operations and the sliding block (21, 21a, 21b) is formed on the casing (12).

13. Dispenser as claimed in any one of Claims 1 to 12, **characterised in that** at least one blocking means (27) is provided preventing partial operations of the operating member (14), provided that a minimum operating force is not applied to the operating member (14).

14. Dispenser as claimed in any one of the preceding Claims, **characterised in that** the container is the ampoule (17) of a thrust piston cylinder which is sealed by means of a plug (18), wherein a discharge channel is formed on the casing and issues into a discharge opening (32) which terminates at its side remote form the discharge opening in a cannula (20) which penetrates the plug (18) during the first partial operation, wherein during each partial operation the plug (18) is moved into the ampoule by a predetermined amount in the direction of the base of the ampoule (17) and a predetermined volume of medium is displaced into the discharge channel.

## Revendications

1. Distributeur pour l'application, le cas échéant par pulvérisation, d'un produit, en particulier liquide, à partir d'un récipient (17), où l'application du produit est réalisée, suivant des charges partielles définies, par une succession d'actionnements partiels manuels d'un élément d'actionnement (14), dans une direction d'actionnement, où il est prévu à chaque fois, entre deux actionnements partiels de l'élément d'actionnement (14), un actionnement d'inversion, par mouvement relatif, qui se produit automatiquement après exécution d'un actionnement partiel où, au cours de l'actionnement partiel, un accumulateur de force (23) est soumis à une tension initiale, accumulateur de force qui, après achèvement de l'actionnement partiel, déclenche l'actionnement d'inversion,
**caractérisé en ce que** l'accumulateur de force (23) est un ressort agissant de façon transversale par rapport à la direction d'actionnement.

2. Distributeur selon la revendication 1, **caractérisé en ce que** l'élément d'actionnement (14), après chaque actionnement partiel, peut être déplacé dans une position initiale d'actionnement, par l'action de l'accumulateur de force (23).

3. Distributeur selon la revendication 1 ou 2, **caractérisé en ce que** le récipient (17) est disposé dans une enveloppe (12) où, au cours de chaque actionnement partiel, le mouvement relatif se produit entre l'enveloppe (12) et l'élément d'actionnement (14), et cette rotation relative est déterminée par un guide de coulisse.

4. Distributeur selon la revendication 3, **caractérisé en ce que** le guide de coulisse est réalisé par au moins une glissière de coulisse (22, 22a, 22b) et par un élément coulissant (21, 21a, 21b) guidé à l'intérieur de la glissière de coulisse.

5. Distributeur selon la revendication 4, **caractérisé en ce qu'**un manchon (13) est disposé entre l'enveloppe (12) et le récipient (17), où le manchon (13) présente soit la glissière de coulisse (22, 22a, 22b) au moins au nombre de un, soit l'élément coulissant (21, 21a, 21b) guidé à l'intérieur de la glissière de coulisse, où le manchon est disposé en étant fixe dans sa position, soit par rapport à l'enveloppe (12), soit par rapport au récipient (17).

6. Distributeur selon la revendication 5, **caractérisé en ce que** le manchon (13), dans la direction de l'actionnement partiel, est disposé en étant fixe dans sa position, soit par rapport à l'enveloppe (12), soit par rapport au récipient (17) et, dans la direction de l'actionnement d'inversion, mobile par rapport à ces éléments.

7. Distributeur selon l'une quelconque des revendications 3 à 6, **caractérisé en ce que** le guide de coulisse est formé par deux glissières de coulisse (22a, 22b) comprenant chacune un élément coulissant (21a, 21b), où la première glissière de coulisse (21a) présente une courbe de glissière étagée correspondant aux actionnements partiels, où une branche de ressort (23), servant d'accumulateur de force et pouvant être poussée transversalement hors de l'espace de déplacement de l'élément coulissant (21a), pénètre dans des parties de glissière de coulisse correspondant à des actionnements partiels où, à la fin de l'actionnement partiel, il se produit un mouvement transversal de l'élément coulissant (21a) sortant de la partie de glissière de coulisse de l'actionnement partiel effectué et passant dans la partie de glissière de coulisse de l'actionnement partiel suivant.

8. Distributeur selon la revendication 7, **caractérisé en ce que** la branche de ressort (23) est configurée de manière telle, qu'elle forme un blocage de recul pour l'élément coulissant (21a).

9. Distributeur selon l'une quelconque des revendications 3 à 8, **caractérisé en ce que** le guide de coulisse est formé par deux glissières de coulisse (22a, 22b), où seulement l'une des glissières de coulisse (21a) présente des branches de ressort (23).

10. Distributeur selon l'une quelconque des revendications 5 à 9, **caractérisé en ce que** le manchon (13) présente la glissière à coulisse (22, 22a, 22b) au moins au nombre de un.

11. Distributeur selon l'une quelconque des revendications 3 à 10, **caractérisé en ce que** le manchon (13), dans la direction d'actionnement des actionnements partiels de l'élément d'actionnement (14), est disposé en étant fixe dans sa position par rapport à l'enveloppe (12), et l'élément coulissant (21, 21a, 21b) est configuré sur l'élément d'actionnement (14).

12. Distributeur selon l'une quelconque des revendications 3 à 10, **caractérisé en ce que** le manchon, dans la direction d'actionnement des actionnements partiels, est disposé en étant fixe dans sa position par rapport au récipient (17), et l'élément coulissant (21, 21a, 21b) est configuré sur l'enveloppe (12).

13. Distributeur selon l'une quelconque des revendications 1 à 12, **caractérisé en ce qu'**il est prévu au moins un moyen de blocage (27) qui empêche des actionnements partiels de l'élément d'actionnement (14), dans la mesure où une force d'actionnement minimum n'est pas appliquée sur l'élément d'actionnement (14).

14. Distributeur selon l'une quelconque des revendications précédentes, **caractérisé en ce que** le récipient est l'ampoule (17) d'un cylindre à piston-poussoir, ampoule qui est obturée par un bouchon (18), où un canal d'application est configuré sur l'enveloppe, canal d'application qui débouche dans une ouverture d'application (32) et qui, au niveau de son côté placé à l'opposé de l'ouverture d'application, se termine dans une canule (20) qui, au cours du premier actionnement partiel, perce le bouchon (18), où le bouchon (18), au cours de chaque actionnement partiel, est déplacé à l'intérieur de l'ampoule, suivant une distance prédéfinie, en direction du fond de l'ampoule (17), et, dans le canal d'application, déplace un volume de produit prédéfini.
